# EUROPEAN PATENT SPECIFICATION

(11) **EP 2 926 867 B1**
(45) Date of publication and mention of the grant of the patent: **01.02.2017**
(21) Application number: 14163090.5
(22) Date of filing: 01.04.2014
(51) Int. Cl.: A61Q 19/00, A61K 8/34, A61K 8/36

(54) **Peeling compositions comprising trichloroacetic acid (tca) and phenol for reducing the burning pain caused by the tca peeling**
Peelingzubereitungen mit Trichloressigsäure (tca) und Phenol zur Reduzierung des brennenden Schmerzes durch das Trichloressigsäure-Peeling
Compositions de décollement comprenant de l'acide trichloroacétique (TCA) et du phénol pour réduire la douleur ardente provoquée par le décollement avec le TCA

(43) Date of publication of application: 07.10.2015
(73) Proprietor: Deprez, Philippe, 17486 Girona (ES)
(72) Inventor: Deprez, Philippe, 17486 Girona (ES)
(74) Representative: Isern-Jara, Nuria

(56) References cited:
- EP-A2- 1 297 831
- WO-A1-97/09963
- WO-A2-2006/077156
- US-A- 5 874 463
- US-A1- 2005 287 096

## Description

### BACKGROUND OF THE INVENTION

Trichloroacetic acid (TCA) peelings are medical dermatological treatments, to be applied by trained physicians only, on the skin of the patients, with the main goal of treating skin photo ageing, fine lines, pigmentations, acne.

Nowadays, a wide range of facial rejuvenation techniques are available: more-or-less aggressive peelings, dermal fillers for wrinkle treatments, laser sessions for certain indications, lipofilling. The chemical peeling is fully considered a part of the current techniques, which overcome the time: historically, chemical peelings using alpha hydroxy acids were already used by the ancient Egyptians. The peeling is to be applied on the surface of the skin; it is a solution which temporarily modifies the pH to obtain an exfoliative action which will cause skin regeneration.

Chemical peelings are skin treatments that are designed to visibly improve the structure of the treated tissues by external application of different acids solutions. Not only may it lead to a simple acceleration of natural phenomena of exfoliation, but also to a complete destruction of the epidermis and a more- or-less important part in the dermis, mainly by coagulation or lysis of proteins. The effect of any peeling extends directly or indirectly, at varying degrees, to the dermis, where the more or less important regeneration processes are induced by the used molecules and their application protocol. These chemical peelings are appealing to aesthetic physicians, dermatologists and surgeons, therefore these peelings meet the market's needs as they are safe, easy to use and the package is well adapted for one or several applications.

The peeling efficacy has been demonstrated by medical publications. The TCA Peeling, in itself, represents no innovation, since TCA peelings were already described in British journal of dermatology in 1926. The phenol peeling is also known, as it is used since the 1920's.

The present invention is based on the fact that the inventors have found that by mixing a small percentage of phenol with the TCA in certain concentrations, the burning pain induced by the Trichloracetic acid (TCA) is substantially reduced in a few seconds. This technique has never been described in medical literature.

Trichloracetic acid peeling is actually present in the market:
Many TCA solutions are present in the market. German literature reports that already in 2004, more than 45 different TCA solutions were offered in the European market. These are very old medical treatments, some of which were already known at the time of the Mesopotamians and the most recent ones were discovered at the end of the 19th century. So there is a tremendous amount of publications and common experiences that helped to progressively refine the mode of use, formulations, and the indications to avoid any complications of these treatments.

TCA peeling is used to coagulate skin proteins, at the same time coagulating the noble structures of cells membranes. Cells are therefore subjected to drying and are replaced by new cells produced by the skin during the healing and "peeling" process, In the known formulations, the concentrations of TCA in the formulations for the TCA peelings range from 10 up to 80%.

On the other hand, phenol peelings are likewise present in the market: Phenol peelings are reported since the 1920's as average, after the World War I. Many types of formulations have been published and some phenol peelings are actually marketed by several companies.

Likewise the TCA peelings, the phenol peelings are used to coagulate the skin proteins, coagulating at the same time the noble structures of cells membranes. Cells therefore are subjected to drying and are replaced by new cells produced by the skin during the healing and "peeling" process. Phenol coagulates the cells faster and deeper as compared to TCA. In the known formulations, the concentrations of phenol in the formulations for the phenol peelings range from 30 up to 65%.

Finally, some formulas can be found in the market that mix TCA and Phenol; for example Easy Phen Light (Skin Tech)® mixes 15% TCA and 30% of phenol. However, the compositions according to the invention have an important difference with this formulation:
Actually, TCA is added to phenol for a clear reason: obtaining a better result with lower phenol concentrations. The action of TCA allows disrupting the skin permeability, and disrupted skin permeability allows a deeper and faster phenol penetration. As a consequence, better results are obtained with a lower phenol concentration, and having the additional advantage of reducing the phenol toxicity. Indeed, TCA is a non-toxic molecule when applied on the skin, but phenol is a toxic molecule even by topical application. A reduced concentration allows reducing the potential toxicity of phenol.

A few medical publications describe the use of phenol at 80% as an anaesthetic before placing surgically ear drums. One publication describes the use of a 20-25% phenol solution before a skin abrasion. Concentrations lower than 10% have not been described to obtain an anesthetic effect.

Other types of TCA + phenol mixtures have been used with the aim to increase the power of the peeling, to increase the penetration of the chemical substances.

Nevertheless, no other mixture of a normal concentration of TCA with a minimal concentration of phenol with the claim to reduce the pain linked with the TCA application has been found in the market or in the litterature.

### SUMMARY OF THE INVENTION

When applying a TCA peeling solution onto the face, the patient feels a strong burning, stinging sensation, as long as the peeling solution is kept active on the skin or as long as the last TCA molecule terminates its coagulating effect. Only at this moment the pain slowly disappears. Pain is often a limiting factor for people who could not undergo the treatment by fear of the burning sensation.

The present inventors have found a solution to this pain: Mixing a little percentage of phenol together with a TCA solution makes the application of the TCA solution much less painful when the TCA concentrations are in the range of 10-25%. All the concentrations described are in weight/weigh or wt%.

The burning sensation of higher TCA concentration cannot be lowered by adding phenol to the solution.

The concentration of phenol to be added to a TCA peel solution concentrated at 10-25% wt% is comprised between 0,5 and 10% wt%

### DETAILED DESCRIPTION OF THE INVENTION

In a first aspect, the invention is directed to formulations useful in lowering the burning pain that appears immediately after the application of a TCA solution on the skin having the following composition:
TCA concentration between 10 - 25% and
Phenol concentration: between 0.5 - 10 %,

The rest of components, being water and pharmaceutically acceptable excipients.

In a second aspect, the invention is directed to the use of such composition as a medicament, in particular as a medicament to lower the burning pain on a skin being subjected to a TCA peeling.

In preferred embodiments, by mixing 0.5% - 10% of phenol with a 8-25% TCA solution makes the application of the TCA solution much less painful as compared to the same concentration of TCA without phenol. However, the burning sensation of TCA concentrations higher than 25% cannot be lowered by adding phenol to the solution.

The concentration of phenol to be added to a TCA peel is comprised between 0.5 and 10%. Phenol is usually used for peeling at average concentrations of 30-80%

### ILLUSTRATIVE EXAMPLES

Any combination can be possible between TCA (8 - 25%) and phenol (0.5 - 10%)
Working, illustrative examples are the following:
TCA 12% + Phenol 2%
TCA 15% + phenol 5%
TCA 25% + phenol 10%

However, all the above embodiments can be taken in combination with each other, so that all combinations are possible.

The efficacy of the pain control of this formulation has been controlled and confirmed in experimental tests carried out by the inventors.

## Claims

1. A formulation comprising tricholoracetic acid (TCA) in a concentration range of 8-25 wt% and phenol in a concentration range of 0,5-10 wt%, the rest of components being water and pharmaceutically acceptable excipients.

2. The formulation according to claim 1, wherein the TCA concentration is in the range of 10-25 wt% and the phenol concentration is in the range of 0.5-10 wt%.

3. The formulation according to claim 1, where the TCA concentration is about 12 wt% and the phenol concentration is about 2 wt%.

4. The formulation according to claim 1, wherein the TCA concentration is about 15 wt% and the phenol concentration is about 5 wt%.

5. The formulation according to claim 1, wherein the TCA concentration is about 25 wt% and the phenol concentration is about 10 wt%.

6. The formulation according to any one of previous claims for use as a medicament.

7. The formulation according to any one of claims 1-5 for use as a medicament to reduce the burning pain caused by the TCA peeling.

8. The formulation according to any one of claims 1-5 for use as a peeling solution.

## Patentansprüche

1. Enthalt von der Formulierung: Tricholoracetic Säure (TCA) in einen Konzentrationsbereich von 8-25 wt % und Phenol in einen Konzentrationsbereich von 0.5-10 wt %, Rest von Bestandteilen entschließt, Wasser und zugelaste pharmazeutische Komponenten.

2. Die Formulierung nach Anspruch 1 worin die TCA Konzentration ist im Bereich von 10-25 wt %, und die Phenol-Konzentration ist in den Bereich von 0.5-10 wt %.

3. Die Formulierung nach den Anspruch 1, worin die TCA Konzentration ist in den Bereich von 12 wt% und die Phenol-Konzentration ist in den Bereich von 2wt%.

4. Die Formulierung nach den Anspruch 1, worin die TCA Konzentration ist in den Bereich von 15 wt% und die Phenol-Konzentration ist in den Bereich von 5wt%.

5. Die Formulierung nach den Anspruch 1, worin die TCA Konzentration ist in den Bereich von 25 wt% und die Phenol-Konzentration ist in den Bereich von 10wt%.

6. Die Formulierung von allen vorherigen Ansprüchen ist geeignet zum Gebrauch als ein Medikament.

7. Die Formulierung von den Ansprüchen 1-5 ist zum Gebrauch als Medikament um den Brennschmerz, erregt durch das TCA peeling zu lindern.

8. Die Formulierung von irgend von den einzellden Ansprüchen 1-5, gebraucht als Peeling.

## Revendications

1. Formulation comprenant de l'acide tricholoracétique (TCA) à une concentration comprise entre 8-25 % du poids total et du phénol une concentration comprise entre 0,5 à 10 % du poids total, le reste des composants étant de l'eau et des excipients pharmaceutiquement acceptables.

2. Formulation selon la revendication 1, dans laquelle la concentration en TCA est comprise entre 10 à 25% du poids total et la concentration en phénol est comprise entre 0,5 à 10% du poids total.

3. Formulation selon la revendication 1, dans laquelle la concentration en TCA est d'environ 12% du poids total et la concentration en phénol est d'environ 2% du poids total.

4. Formulation selon la revendication 1, dans laquelle la concentration en TCA est d'environ 15% du poids total et la concentration en phénol est d'environ 5% du poids total.

5. Formulation selon la revendication 1, dans laquelle la concentration en TCA est d'environ 25% du poids total et la concentration en phénol est d'environ 10% du poids total.

6. Formulation incluant les revendications précédentes de manière indépendante ou conjointement pour une utilisation en tant que médicament.

7. Formulation incluant les revendications 1 à 5 de manière indépendante ou conjointement, destinée à être utilisée comme médicament pour réduire la douleur causée par un peeling au TCA.

8. Formulation incluant les revendications 1 à 5 de manière indépendante ou conjointement, destinée à être utilisée comme solution de peeling.
